# EUROPEAN PATENT SPECIFICATION

(11) **EP 3 245 984 B1**
(45) Date of publication and mention of the grant of the patent: **07.11.2018**
(21) Application number: 15877990.0
(22) Date of filing: 08.12.2015
(51) Int. Cl.: A61F 2/07, A61F 2/90, A61F 2/04

(54) **STENT AND STENT GRAFT**
STENT UND STENTPROTHESE
ENDOPROTHÈSE ET GREFFE D'ENDOPROTHÈSE

(30) Priority: 13.01.2015 JP 2015004541
(43) Date of publication of application: 22.11.2017
(73) Proprietor: Japan Lifeline Co., Ltd., Tokyo 140-0002 (JP)
(72) Inventor: NAKANO, Eiichi, Tokyo 140-0002 (JP); KOBAYASHI, Fumiaki, Tokyo 140-0002 (JP)
(74) Representative: Grünecker Patent- und Rechtsanwälte PartG mbB
(86) International application number: PCT/JP2015/084453
(87) International publication number: WO 2016/114039

(56) References cited:
- EP-A1- 2 918 251
- JP-A- H1 033 692
- JP-A- 2005 523 107
- JP-A- 2006 026 329
- JP-A- 2006 026 329
- JP-A- 2010 524 568
- JP-A- 2014 217 487
- JP-A- 2014 217 487

## Description

### Technical Field

The present invention relates to a stent and a stent graft to be placed in an internal tubular organ such as a digestive tract so as to prevent the constriction or occlusion of the tubular organ.

### Background Art

A stent (a digestive tract stent) to be placed in a digestive tract is used for expanding the lumen of the digestive tract that has been constricted by a tumor.

Such a digestive tract stent is required to exert a great diameter-expanding force for fully expanding the constricted part, to exhibit good diameter reducibility for smooth insertion into a delivery sheath, and to exhibit good followability along curved parts of the digestive tract.

On the other hand, after the digestive tract stent is placed, tumor tissues increasing with the growth of the tumor may enter the lumen of the digestive stent through the mesh of the stent and may reconstrict the digestive tract.

To prevent such reconstriction of the digestive tract, the mesh of the stent is desired to be made as fine as possible (to make the spaces between lines of wire forming the mesh as small as possible).

However, if the mesh of the stent is made finer, the number of mesh spaces that are provided next to one another increases in the peripheral direction and in the axial direction. Consequently, the number of connections between lines of wire (for example, the number of interlaced parts where bent parts of different lines of wire are connected) increases.

With such an increase in the number of connections (interlaced parts) that are at the same position in the axial direction and are next to one another in the peripheral direction, the diameter reducibility of the stent tends to be deteriorated.

Furthermore, with an increase in the number of connections (interlaced parts) that are at the same position in the peripheral direction and are next to one another in the axial direction, the followability along curved parts of the tubular organ tends to be deteriorated. JP2014 217487 discribes a slent formed by weaving a single wire.

A stent disclosed by PTL 1 listed below is formed as follows. After a first cylindrical lattice structure is formed with a wire (a first member), a second lattice structure having the same shape is formed with another wire (a second member) such that the second lattice structure is shifted from the first cylindrical lattice structure by 1/4 pitch in the peripheral direction.

In such a stent, each of the mesh spaces in one of the cylindrical lattice structures can be divided into four sections by the lines of wire forming the other cylindrical lattice structure. Hence, the mesh of the stent can be made finer (see Fig. 13 of PTL 1).

### Citation List

### Patent Literature

PTL 1: Japanese Unexamined Patent Application Publication (Translation of PCT Application) No. 2009-501049

### Summary of Invention

### Technical Problem

In the stent disclosed by PTL 1, however, the number of interlaced parts that are next to one another in the axial direction at each of some peripheral-direction positions (for example, each of positions y1, y3, y7, y9, and y13 in Fig. 13 of PTL 1) and the number of interlaced parts that are next to one another in the peripheral direction at each of some axial-direction positions (for example, each of positions x1 and x9 in Fig. 13 of PTL 1) are each twice that of a stent including only the first cylindrical lattice structure.

Hence, the stent disclosed by PTL 1 has poor curved-part followability and poor diameter reducibility.

The present invention has been conceived in view of the above circumstances.

An object of the present invention is to provide a stent having a fine mesh with no deterioration in the diameter reducibility and the curved-part followability that are required of the stent and in which the reconstriction caused by tumor tissues that may enter the lumen of the stent placed in a tubular organ can therefore be prevented. Solution to Problem
(1) A stent according to the present invention is a stent formed with at least one wire woven into a cylindrical shape. The stent includes:
   a first woven structure including a plurality of peripheral-direction units that are arranged next to one another in an axial direction, the peripheral-direction units each having a plurality of mesh spaces provided next to one another in a peripheral direction, the peripheral-direction units each including a first loop and a second loop, the first loop having bent parts and linear parts formed by drawing the wire in the peripheral direction while folding the wire alternately to a left side and to a right side, the second loop having bent parts and linear parts formed by drawing the wire in the peripheral direction continuously from the first loop while folding the wire alternately to the left side and to the right side such that the second loop becomes out of phase with the first loop by 1/2 pitch; and
   a second woven structure including a plurality of peripheral-direction units that are arranged next to one another in the axial direction in correspondence with the peripheral-direction units of the first woven structure, the peripheral-direction units each having a plurality of mesh spaces provided next to one another in the peripheral direction, the peripheral-direction units each including a first loop and a second loop, the first loop having bent parts and linear parts formed by drawing the wire in the peripheral direction while folding the wire alternately to the left side and to the right side at the same pitch length as the peripheral-direction units of the first woven structure and at a smaller amplitude than the peripheral-direction units of the first woven structure, the second loop having bent parts and linear parts formed by drawing the wire in the peripheral direction continuously from the first loop while folding the wire alternately to the left side and to the right side such that the second loop becomes out of phase with the first loop by 1/2 pitch,
   wherein, in a region where each two of the peripheral-direction units of the first woven structure overlap each other, the bent parts of one of the two peripheral-direction units are connected to the bent parts or wire intersections of the other peripheral-direction unit,
   wherein the peripheral-direction units of the second woven structure are each shifted from a corresponding one of the peripheral-direction units of the first woven structure by 1/4 pitch in the peripheral direction and are each woven together with the corresponding one of the peripheral-direction units of the first woven structure, and
   wherein, regarding adjacent ones of the peripheral-direction units of the second woven structure, the bent parts of one of the adjacent peripheral-direction units are connected to neither the bent parts nor wire intersections of the other peripheral-direction unit.

In the stent configured as above, the peripheral-direction units of the second woven structure are each shifted from a corresponding one of the peripheral-direction units of the first woven structure by 1/4 pitch in the peripheral direction. Hence, the mesh spaces of the first woven structure can each be divided into four sections by lines of the wire forming the second woven structure. Therefore, the mesh of the stent can be made finer.

Furthermore, the peripheral-direction units of the second woven structure are each shifted from a corresponding one of the peripheral-direction units of the first woven structure by 1/4 pitch in the peripheral direction. Therefore, the connections at the bent parts of the peripheral-direction units of the first woven structure are not provided at the same peripheral-direction positions as and are not aligned in the axial direction with the connections at the bent parts of the peripheral-direction units of the second woven structure. Hence, the deterioration in the curved-part followability that may be caused by the doubling of the number of connections provided next to one another at each peripheral-direction position can be avoided.

Furthermore, the peripheral-direction units of the second woven structure have a smaller amplitude than the peripheral-direction units of the first woven structure. Therefore, the connections at the bent parts of the peripheral-direction units of the first woven structure are not provided at the same axial-direction positions as and are not aligned in the peripheral direction with the connections at the bent parts of the peripheral-direction units of the second woven structure. Hence, the deterioration in the diameter reducibility that may be caused by the doubling of the number of connections provided next to one another at each axial-direction position can be avoided.

Furthermore, regarding adjacent ones of the peripheral-direction units of the second woven structure, the bent parts of one of the adjacent peripheral-direction units are connected to neither the bent parts nor wire intersections of the other peripheral-direction unit. Therefore, the curved-part followability and the diameter reducibility can be made better than in a connected case.
(2) In the stent according to the present invention, it is preferable that the plurality of peripheral-direction units of the first woven structure be arranged next to one another in the axial direction by straightly drawing, without bending, an end of the loop forming one of the peripheral-direction units and by forming a subsequent one of the peripheral-direction units at a position shifted from the preceding peripheral-direction unit in the axial direction, and
   that the plurality of peripheral-direction units of the second woven structure be arranged next to one another in the axial direction by straightly drawing, without bending, an end of the loop forming one of the peripheral-direction units and by forming a subsequent one of the peripheral-direction units at a position shifted from the preceding peripheral-direction unit in the axial direction by an amount equal to an amount of shift between adjacent ones of the peripheral-direction units of the first woven structure.
   Thus, all of the peripheral-direction units of the second woven structure correspond to the peripheral-direction units of the first woven structure, respectively.
(3) In the stent according the present invention, it is preferable that the peripheral-direction units of the second woven structure have an amplitude of 25 to 95% of an amplitude of the peripheral-direction units of the first woven structure.
(4) In the stent according to the present invention, it is preferable that the peripheral-direction units of the first woven structure be each shifted from a preceding one of the peripheral-direction units by approximately 1/2 pitch (1/2 of the amplitude) in the axial direction and by approximately 1/4 pitch in the peripheral direction, and, in parts where adjacent ones of the peripheral-direction units of the first woven structure overlap each other, the bent parts of one of the adjacent peripheral-direction units be connected to the wire intersections of the other peripheral-direction unit.
(5) In the stent described in (4), it is preferable that the bent parts of each of the peripheral-direction units of the second woven structure be not connected to the bent parts of any peripheral-direction unit of the first woven structure that is adjacent to a corresponding one of the peripheral-direction units of the first woven structure.
(6) A stent graft according to the present invention includes the stent according to the present invention, and a graft that covers an inner periphery and/or an outer periphery of the stent.

### Advantageous Effects of Invention

The stent according to the present invention can have a fine mesh with no deterioration in the diameter reducibility and the followability along curved parts of the tubular organ that are required of the stent. Therefore, the reconstriction caused by tumor tissues that may enter the lumen of the stent placed in the tubular organ can be prevented.

### Brief Description of Drawings

[Fig. 1] Fig. 1 schematically illustrates a part of a configuration of a stent according to the present invention.
[Fig. 2] Fig. 2 is a development of a relevant part of a stent according to a first embodiment of the present invention.
[Fig. 3A] Fig. 3A is a development illustrating a step of forming the stent illustrated in Fig. 2.
[Fig. 3B] Fig. 3B is a development illustrating another step of forming the stent illustrated in Fig. 2.
[Fig. 3C] Fig. 3C is a development illustrating yet another step of forming the stent illustrated in Fig. 2.
[Fig. 3D] Fig. 3D is a development illustrating yet another step of forming the stent illustrated in Fig. 2.
[Fig. 3E] Fig. 3E is a development illustrating yet another step of forming the stent illustrated in Fig. 2.
[Fig. 3F] Fig. 3F is a development illustrating yet another step of forming the stent illustrated in Fig. 2.
[Fig. 3G] Fig. 3G is a development illustrating yet another step of forming the stent illustrated in Fig. 2.
[Fig. 3H] Fig. 3H is a development illustrating yet another step of forming the stent illustrated in Fig. 2.
[Fig. 3I] Fig. 3I is a development illustrating yet another step of forming the stent illustrated in Fig. 2.
[Fig. 3J] Fig. 3J is a development illustrating yet another step of forming the stent illustrated in Fig. 2.
[Fig. 3K] Fig. 3K is a development illustrating yet another step of forming the stent illustrated in Fig. 2.
[Fig. 3L] Fig. 3L is a development illustrating yet another step of forming the stent illustrated in Fig. 2.
[Fig. 3M] Fig. 3M is a development illustrating yet another step of forming the stent illustrated in Fig. 2.
[Fig. 4] Fig. 4 is a development of a relevant part of a stent according to a second embodiment of the present invention.
[Fig. 5] Fig. 5 is another development of the stent according to the second embodiment illustrated in Fig. 4 and illustrates differences from the stent according to the first embodiment illustrated in Fig. 2.
[Fig. 6] Fig. 6 is a development of a relevant part of a stent according to a third embodiment of the present invention.
[Fig. 7] Fig. 7 is a development of a relevant part of a stent according to a fourth embodiment of the present invention.

### Description of Embodiments

### <Basic Configuration>

A basic configuration of a stent according to the present invention will now be described in detail.

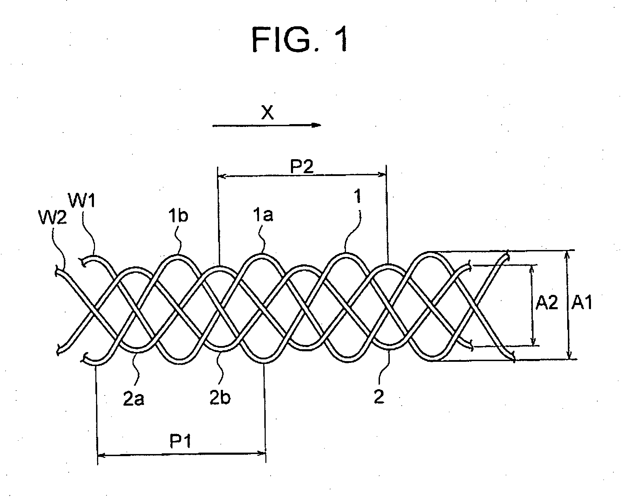

Fig. 1 schematically illustrates a peripheral-direction unit 1 forming a first woven structure and a peripheral-direction unit 2 forming a second woven structure that are woven into the stent according to the present invention.

As illustrated in Fig. 1, the peripheral-direction unit 1 and the peripheral-direction unit 2 have the same pitch length (pitch lengths P1 and P2 in the peripheral direction) but are out of phase with each other by 1/4 pitch with different amplitudes (A1 and A2).

The pitch length (P1 and P2) of the peripheral-direction unit 1 and the peripheral-direction unit 2 is, for example, 12 to 48 mm and preferably 18 to 36 mm.

The amplitude (A1) of the peripheral-direction unit 1 is, for example, 8 to 24 mm and preferably 12 to 20 mm.

The peripheral-direction unit 1 of the first woven structure is formed with a wire W1 and has bent parts and linear parts. A plurality of (five) mesh spaces each defined by lines of the wire W1 are provided next to one another in the peripheral direction (the direction indicated by arrow X).

The peripheral-direction unit 1 includes a first loop 1a formed with the wire W1 drawn in the peripheral direction while being folded alternately to the left side and to the right side at the constant amplitude A1, and a second loop 1b formed with the wire W1 drawn in the peripheral direction continuously from the first loop 1a while being folded alternately to the left side and to the right side at the amplitude A1 in such a manner as to be out of phase with the first loop 1a by 1/2 pitch.

The second loop 1b intersects the linear parts of the first loop 1a and runs alternately over and under the first loop 1a (in such a manner as to be woven together with the first loop 1a).

The peripheral-direction unit 2 of the second woven structure is formed with a wire W2 and has bent parts and linear parts. A plurality of (five) mesh spaces each defined by lines of the wire W2 are provide next to one another in the peripheral direction.

The peripheral-direction unit 2 includes a first loop 2a formed with the wire W2 drawn in the peripheral direction while being folded alternately to the left side and to the right side at the constant amplitude A2 (note that A2 < A1), and a second loop 2b formed with the wire W2 drawn in the peripheral direction continuously from the first loop 2a while being folded alternately to the left side and to the right side in such a manner as to be out of phase with the first loop 2a by 1/2 pitch.

The material of the wires (the wires W1 and W2) forming the stent according to the present invention is preferably metal. In particular, a shape-memory alloy that exerts a shape-memory effect and superelasticity imparted thereto when heated is preferable. Depending on the purpose of use, any of materials such as stainless steel, Ta, Ti, Pt, Au, and W may be employed.

As illustrated in Fig. 1, the pitch length P1 of the peripheral-direction unit 1 and the pitch length P2 of the peripheral-direction unit 2 are equal, but the peripheral-direction unit 1 and the peripheral-direction unit 2 are out of phase with each other by 1/4 pitch. Hence, the mesh spaces of the first woven structure (the peripheral-direction unit 1) can each be divided into four sections by the lines of the wire W2 forming the second woven structure (the peripheral-direction unit 2). Consequently, the mesh can be made finer than that of a stent including only the first woven structure.

Furthermore, as illustrated in Fig. 1, the peripheral-direction unit 2 of the second woven structure is shifted from the peripheral-direction unit 1 of the first woven structure by 1/4 pitch in the peripheral direction. Hence, the bent parts of the peripheral-direction unit 1 are not at the same peripheral-direction positions as the bent parts of the peripheral-direction unit 2, and connections to be formed at the bent parts of the peripheral-direction unit 1 are not provided at the same peripheral-direction positions as (are not concentrated on) connections to be formed at the bent parts of the peripheral-direction unit 2. Therefore, the deterioration in the followability along curved parts of the tubular organ can be avoided.

Furthermore, as illustrated in Fig. 1, the amplitude (A2) of the peripheral-direction unit 2 of the second woven structure is smaller than the amplitude (A1) of the peripheral-direction unit 1 of the first woven structure. Hence, connections to be formed at the bent parts of the peripheral-direction unit 1 are not provided at the same axial-direction positions as (are not concentrated on) connections to be formed at the bent parts of the peripheral-direction unit 2. Therefore, the deterioration in the diameter reducibility of the stent can be avoided.

The ratio of the amplitude A2 of the peripheral-direction unit 2 to the amplitude A1 of the peripheral-direction unit 1 (the ratio A2/A1) is preferably 25 to 95%, and more preferably 40 to 80%. A suitable example is 75%.

If the value of the ratio (A2/A1) is too small, dividing each of the mesh spaces of one of the two woven structures into four sections by the lines of the wire forming the other woven structure results in two relatively small mesh spaces and two relatively large mesh spaces. The two relatively large mesh spaces may allow the passage of tumor tissues, making it difficult to achieve the object of the present invention.

On the other hand, if the value of the ratio (A2/A1) is close to 100%, the axial-direction distance between each of the bent parts of the peripheral-direction units forming the first woven structure and a corresponding one of the bent parts of the peripheral-direction units forming the second woven structure becomes short, leading to a possible deterioration in the diameter reducibility of the stent.

### <First Embodiment>

Now, specific embodiments of the present invention will be described in detail.

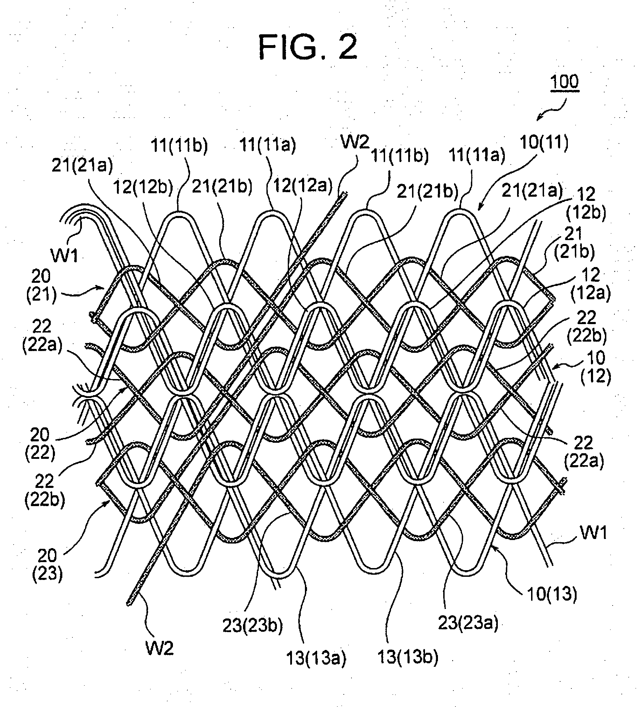

A stent 100 according to a first embodiment illustrated in Fig. 2 includes a first woven structure 10 in which a peripheral-direction unit 11 in a first row, a peripheral-direction unit 12 in a second row, and a peripheral-direction unit 13 in a third row are arranged next to one another in the axial direction while each including parts overlapping parts of one of the other peripheral-direction units that is adjacent thereto; and a second woven structure 20 in which a peripheral-direction unit 21 in a first row, a peripheral-direction unit 22 in a second row, and a peripheral-direction unit 23 in a third row are arranged next to one another in the axial direction while each overlapping none of the other peripheral-direction units that is adjacent thereto.

The peripheral-direction unit 11 in the first row of the first woven structure 10 includes a first loop 11a formed with a wire W1 drawn in the peripheral direction while being folded alternately to the left side and to the right side, and a second loop 11b formed with the wire W1 drawn in the peripheral direction continuously from the first loop 11a while being folded alternately to the left side and to the right side in such a manner as to be out of phase with the first loop 11a by 1/2 pitch.

The second loop 11b intersects the linear parts of the first loop 11a and runs alternately over and under the first loop 11a.

The peripheral-direction unit 12 in the second row of the first woven structure 10 is shifted from the peripheral-direction unit 11 in the first row by 1/2 pitch (1/2 of the amplitude corresponding to the axial-direction length of the peripheral-direction unit 11) in the axial direction and by 1/4 pitch in the peripheral direction.

The peripheral-direction unit 12 includes a first loop 12a formed with the wire W1 drawn in the peripheral direction continuously from the second loop 11b of the peripheral-direction unit 11 in the first row while being folded alternately to the left side and to the right side, and a second loop 12b formed with the wire W1 drawn in the peripheral direction continuously from the first loop 12a while being folded alternately to the left side and to the right side in such a manner as to be out of phase with the first loop 12a by 1/2 pitch.

The second loop 12b intersects the linear parts of the first loop 12a and runs alternately over and under the first loop 12a.

The peripheral-direction unit 12 in the second row that is shifted from the peripheral-direction unit 11 in the first row by 1/2 pitch in the axial direction includes parts that overlap the peripheral-direction unit 11.

The bent parts of the peripheral-direction unit 12 that is shifted from the peripheral-direction unit 11 in the first row by 1/4 pitch in the peripheral direction are connected to the wire intersections of the peripheral-direction unit 11. The wire intersections of the peripheral-direction unit 12 are connected to the bent parts of the peripheral-direction unit 11.

The peripheral-direction unit 13 in the third row of the first woven structure 10 is shifted from the peripheral-direction unit 12 in the second row by 1/2 pitch in the axial direction and by 1/4 pitch in the peripheral direction.

The peripheral-direction unit 13 includes a first loop 13a formed with the wire W1 drawn in the peripheral direction continuously from the second loop 12b of the peripheral-direction unit 12 in the second row while being folded alternately to the left side and to the right side, and a second loop 13b formed with the wire W1 drawn in the peripheral direction continuously from the first loop 13a while being folded alternately to the left side and to the right side in such a manner as to be out of phase with the first loop 13a by 1/2 pitch.

The second loop 13b intersects the linear parts of the first loop 13a and runs alternately over and under the first loop 13a.

The peripheral-direction unit 13 in the third row that is shifted from the peripheral-direction unit 12 in the second row by 1/2 pitch in the axial direction includes parts that overlap the peripheral-direction unit 12.

The bent parts of the peripheral-direction unit 13 that is shifted from the peripheral-direction unit 12 in the second row by 1/4 pitch in the peripheral direction are connected to the wire intersections of the peripheral-direction unit 12. The wire intersections of the peripheral-direction unit 13 are connected to the bent parts of the peripheral-direction unit 12.

The peripheral-direction unit 21 in the first row of the second woven structure 20 includes a first loop 21a formed with a wire W2 drawn in the peripheral direction while being folded alternately to the left side and to the right side in such a manner as to have the same pitch length as the peripheral-direction unit 11 of the first woven structure 10 that corresponds thereto and a smaller amplitude than the corresponding peripheral-direction unit 11 and to be shifted from the corresponding peripheral-direction unit 11 by 1/4 pitch in the peripheral direction. The peripheral-direction unit 21 further includes a second loop 21b formed with the wire W2 drawn in the peripheral direction continuously from the first loop 21a while being folded alternately to the left side and to the right side in such a manner as to be out of phase with the first loop 21a by 1/2 pitch.

The second loop 21b intersects the linear parts of the first loop 21a and runs alternately over and under the first loop 21a.

The peripheral-direction unit 21 is woven together with the corresponding peripheral-direction unit 11 of the first woven structure 10. The bent parts of the peripheral-direction unit 21 are connected to (interlaced with) the bent parts of the peripheral-direction unit 12 of the first woven structure 10.

The peripheral-direction unit 22 in the second row of the second woven structure 20 includes a first loop 22a formed with the wire W2 drawn in the peripheral direction while being folded alternately to the left side and to the right side in such a manner as to have the same pitch length as the peripheral-direction unit 12 of the first woven structure 10 that corresponds thereto and a smaller amplitude than the corresponding peripheral-direction unit 12 and to be shifted from the corresponding peripheral-direction unit 12 by 1/4 pitch in the peripheral direction. The peripheral-direction unit 22 further includes a second loop 22b formed with the wire W2 drawn in the peripheral direction continuously from the first loop 22a while being folded alternately to the left side and to the right side in such a manner as to be out of phase with the first loop 22a by 1/2 pitch.

The second loop 22b intersects the linear parts of the first loop 22a and runs alternately over and under the first loop 22a.

The peripheral-direction unit 22 is woven together with the corresponding peripheral-direction unit 12 of the first woven structure 10. The bent parts of the peripheral-direction unit 22 are connected to (interlaced with) the bent parts of the peripheral-direction unit 11 of the first woven structure 10 and are also connected to (interlaced with) the bent parts of the peripheral-direction unit 13 of the first woven structure 10.

The peripheral-direction unit 22 and the peripheral-direction unit 21 do not overlap each other at all. The bent parts of the peripheral-direction unit 22 are connected to neither the bent parts nor the wire intersections of the peripheral-direction unit 21 in the first row.

The peripheral-direction unit 23 in the third row of the second woven structure 20 includes a first loop 23a formed with the wire W2 drawn in the peripheral direction while being folded alternately to the left side and to the right side in such a manner as to have the same pitch length as the peripheral-direction unit 13 of the first woven structure 10 that corresponds thereto and a smaller amplitude than the corresponding peripheral-direction unit 13 and to be shifted from the corresponding peripheral-direction unit 13 by 1/4 pitch in the peripheral direction. The peripheral-direction unit 23 further includes a second loop 23b formed with the wire W2 drawn in the peripheral direction continuously from the first loop 23a while being folded alternately to the left side and to the right side in such a manner as to be out of phase with the first loop 23a by 1/2 pitch.

The second loop 23b intersects the linear parts of the first loop 23a and runs alternately over and under the first loop 23a.

The peripheral-direction unit 23 is woven together with the corresponding peripheral-direction unit 13 of the first woven structure 10. The bent parts of the peripheral-direction unit 23 are connected to (interlaced with) the bent parts of the peripheral-direction unit 12 of the first woven structure 10.

The peripheral-direction unit 23 and the peripheral-direction unit 22 do not overlap each other at all. The bent parts of the peripheral-direction unit 23 are connected to neither the bent parts nor the wire intersections of the peripheral-direction unit 22 in the second row.

The stent 100 according to the first embodiment can be formed by weaving the wire W1 and the wire W2 together in the following procedure, for example.

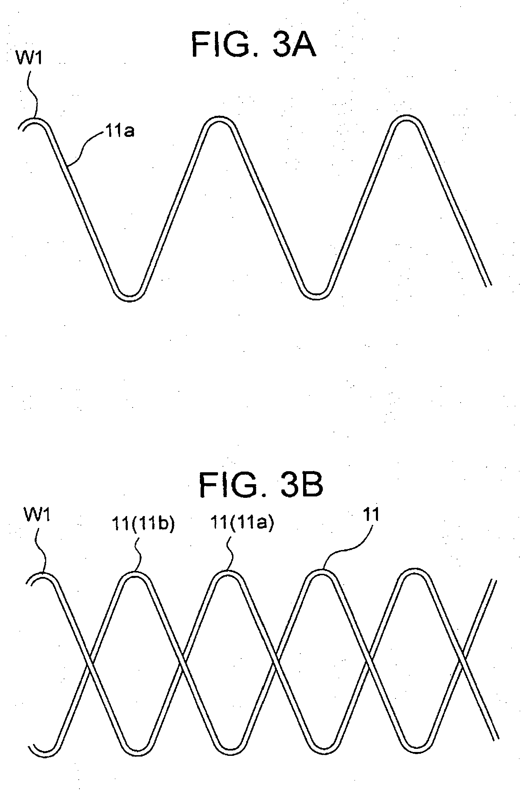

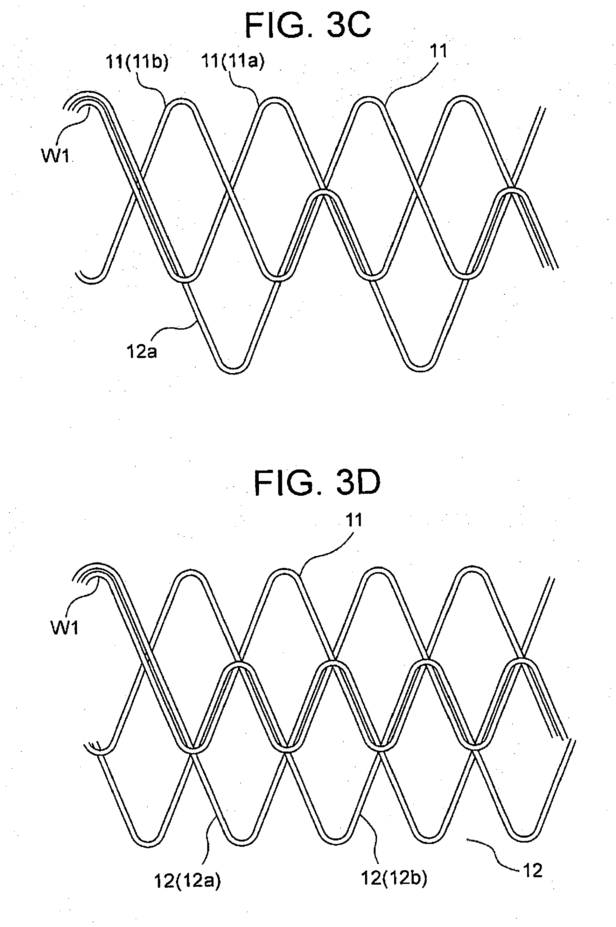

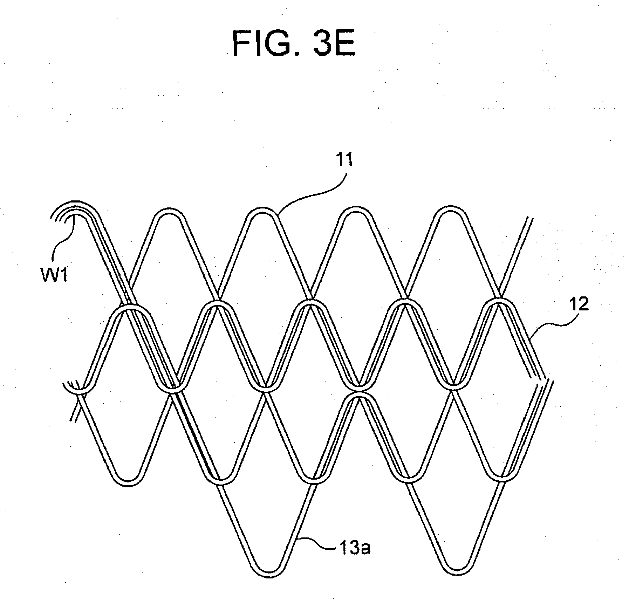

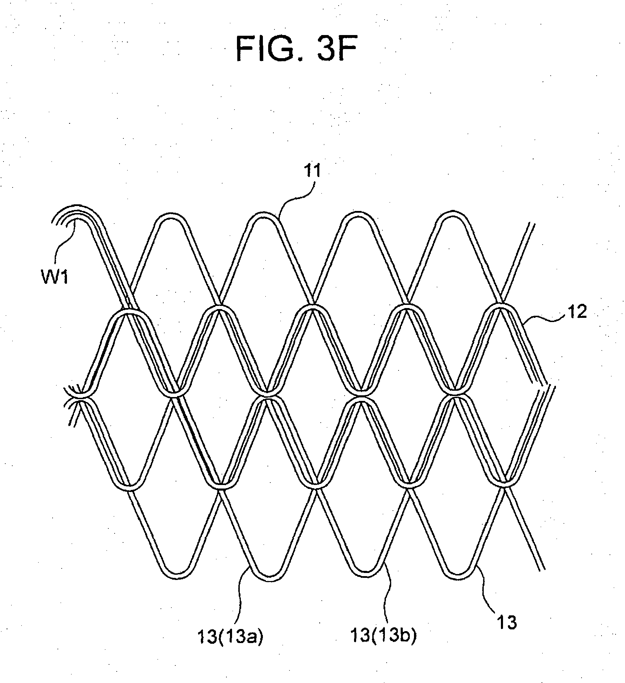

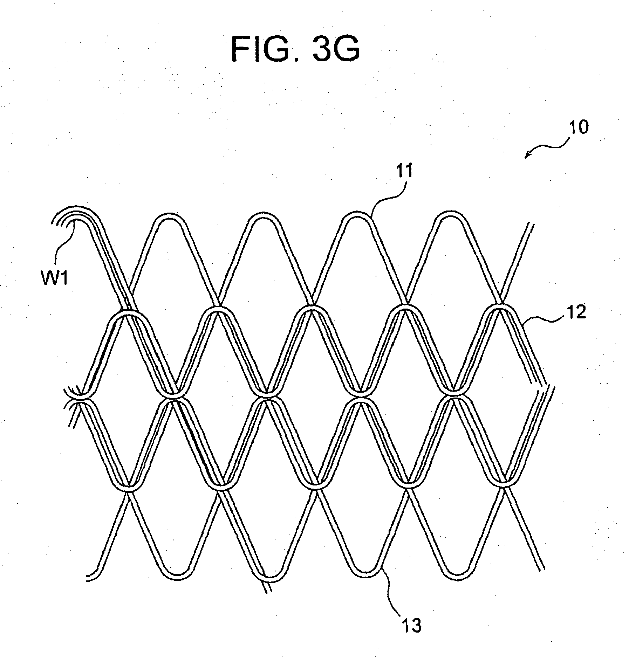

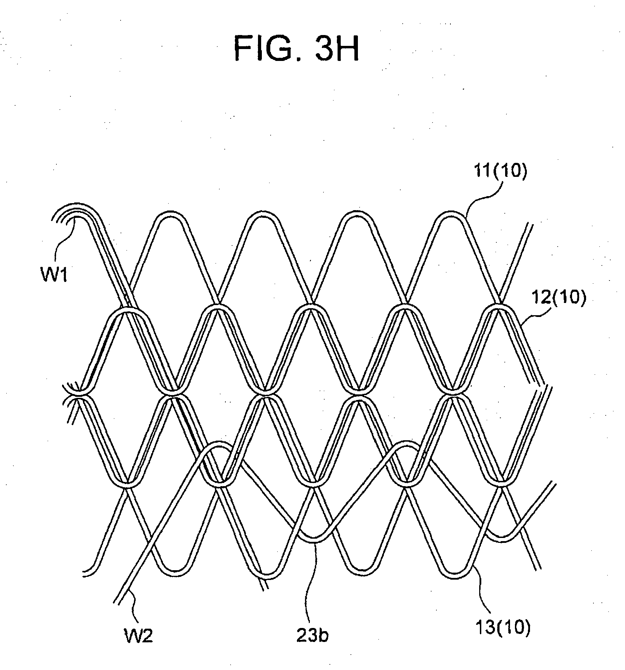

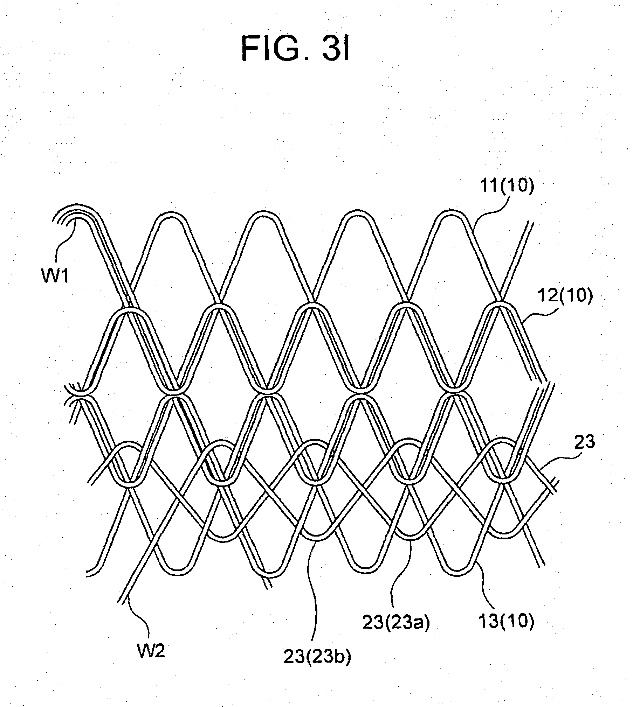

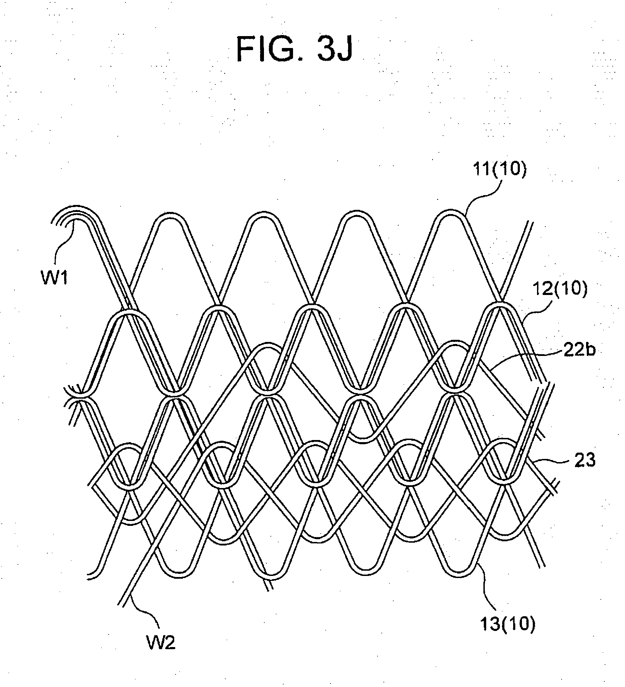

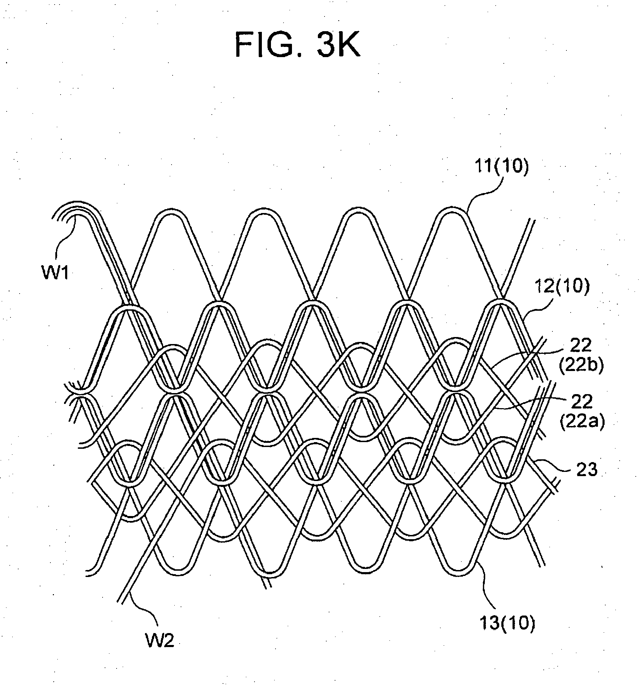

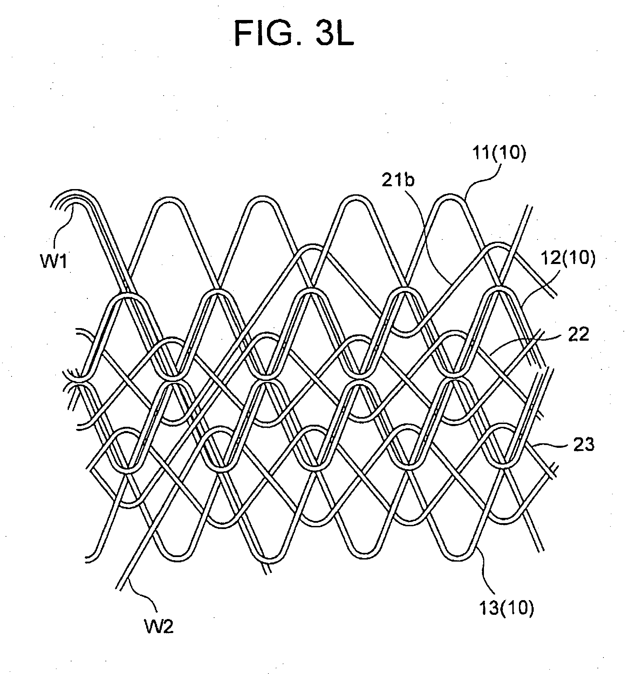

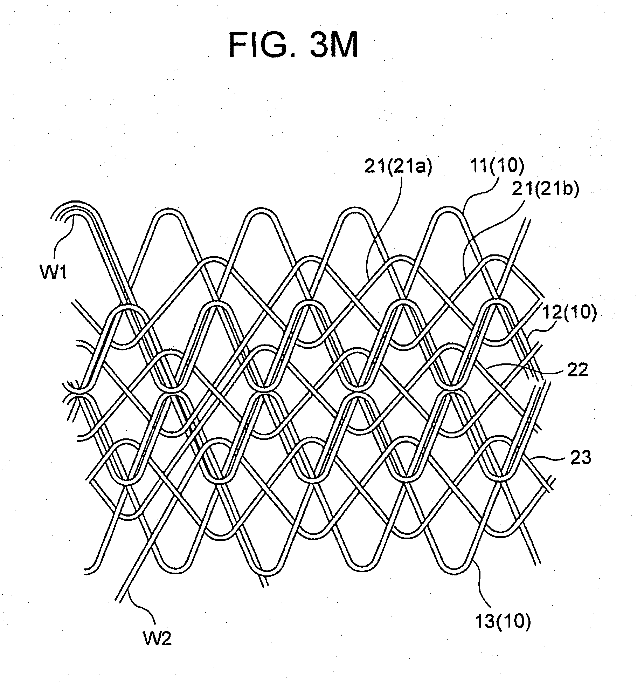

Normally, the weaving process is performed by wrapping the wires around the outer periphery of a mandrel in such a manner as to form predetermined patterns.
(1) A wire W1 is drawn in the peripheral direction while being folded alternately to the left side and to the right side at a constant amplitude, whereby a first loop 11a is formed (see Fig. 3A). After one turn of the wire W1 is formed, the wire W1 is further drawn in the peripheral direction continuously from the first loop 11a while being folded alternately to the left side and to the right side at the same amplitude as the first loop 11a in such a manner as to be woven together with the first loop 11a with a phase difference of 1/2 pitch from the first loop 11a, whereby a second loop 11b is formed. Thus, a peripheral-direction unit 11 in a first row in which a plurality of mesh spaces are provided next to one another in the peripheral direction is formed (see Fig. 3B).
(2) Subsequently, the end of the second loop 11b forming the peripheral-direction unit 11 is drawn, without being bent, by 1/2 pitch (1/2 of the amplitude) in the axial direction and by 1/4 pitch in the peripheral direction. Then, the wire W1 is drawn in the peripheral direction while being folded alternately to the left side and to the right side, whereby a first loop 12a is formed (see Fig. 3C). After one turn of the wire W1 is formed, the wire W1 is further drawn in the peripheral direction continuously from the first loop 12a while being folded alternately to the left side and to the right side in such a manner as to be woven together with the first loop 12a with a phase difference of 1/2 pitch from the first loop 12a, whereby a second loop 12b is formed. Thus, a peripheral-direction unit 12 in a second row in which a plurality of mesh spaces are provided next to one another in the peripheral direction is formed (see Fig. 3D).
(3) Subsequently, the end of the second loop 12b forming the peripheral-direction unit 12 is drawn, without being bent, by 1/2 pitch in the axial direction and by 1/4 pitch in the peripheral direction. Then, the wire W1 is drawn in the peripheral direction while being folded alternately to the left side and to the right side, whereby a first loop 13a is formed (see Fig. 3E). After one turn of the wire W1 is formed, the wire W1 is further drawn in the peripheral direction continuously from the first loop 13a while being folded alternately to the left side and to the right side in such a manner as to be woven together with the first loop 13a with a phase difference of 1/2 pitch from the first loop 13a, whereby a second loop 13b is formed. Thus, a peripheral-direction unit 13 in a third row in which a plurality of mesh spaces are provided next to one another in the peripheral direction is formed (see Fig. 3F).
   Consequently, a first woven structure 10 is formed in which the peripheral-direction unit 11 in the first row, the peripheral-direction unit 12 in the second row, and the peripheral-direction unit 13 in the third row are arranged next to one another in the axial direction while each including parts overlapping one of the other peripheral-direction units that is adjacent thereto (see Fig. 3G).
(4) Subsequently, a wire W2 is drawn in the peripheral direction while being folded alternately to the left side and to the right side at the same pitch length as the peripheral-direction unit 13 of the first woven structure 10 but at a smaller amplitude than the peripheral-direction unit 13 in such a manner as to be woven together with the peripheral-direction unit 13, whereby a second loop 23b is formed (see Fig. 3H). After one turn of the wire W2 is formed, the wire W2 is further drawn in the peripheral direction continuously from the second loop 23b while being folded alternately to the left side and to the right side in such a manner as to be woven together with the second loop 23b and the peripheral-direction unit 13 with a phase difference of 1/2 pitch from the second loop 23b, whereby a first loop 23a is formed. Thus, a peripheral-direction unit 23 in a third row (corresponding to the peripheral-direction unit 13) in which a plurality of mesh spaces are provided next to one another in the peripheral direction is formed (see Fig. 3I). The peripheral-direction unit 23 is shifted from the peripheral-direction unit 13 of the first woven structure 10 by 1/4 pitch in the peripheral direction and is woven together with the peripheral-direction unit 13.
(5) Subsequently, the end of the first loop 23a forming the peripheral-direction unit 23 is drawn, without being bent, by an amount corresponding to 1/2 pitch of the peripheral-direction units of the first woven structure 10 (the same as the amount of shift between adjacent ones of the peripheral-direction units of the first woven structure 10) in the axial direction and by 1/4 pitch in the peripheral direction. Then, the wire W2 is drawn in the peripheral direction while being folded alternately to the left side and to the right side at the same pitch length as the peripheral-direction unit 12 of the first woven structure 10 but at a smaller amplitude than the peripheral-direction unit 12 in such a manner as to be woven together with the peripheral-direction unit 12, whereby a second loop 22b is formed (see Fig. 3J). After one turn of the wire W2 is formed, the wire W2 is further drawn in the peripheral direction continuously from the second loop 22b while being folded alternately to the left side and to the right side in such a manner as to be woven together with the second loop 22b and the peripheral-direction unit 12 with a phase difference of 1/2 pitch from the second loop 22b, whereby a first loop 22a is formed. Thus, a peripheral-direction unit 22 in a second row (corresponding to the peripheral-direction unit 12) in which a plurality of mesh spaces are provided next to one another in the peripheral direction is formed (see Fig. 3K). The peripheral-direction unit 22 is shifted from the peripheral-direction unit 12 of the first woven structure 10 by 1/4 pitch in the peripheral direction and is woven together with the peripheral-direction unit 12.
(6) Subsequently, the end of the first loop 22a forming the peripheral-direction unit 22 is drawn, without being bent, by an amount corresponding to 1/2 pitch of the peripheral-direction units of the first woven structure 10 (the same as the amount of shift between adjacent ones of the peripheral-direction units of the first woven structure 10) in the axial direction and by 1/4 pitch in the peripheral direction. Then, the wire W2 is drawn in the peripheral direction while being folded alternately to the left side and to the right side at the same pitch length as the peripheral-direction unit 11 of the first woven structure 10 but at a smaller amplitude than the peripheral-direction unit 11 in such a manner as to be woven together with the peripheral-direction unit 11, whereby a second loop 21b is formed (see Fig. 3L). After one turn of the wire W2 is formed, the wire W2 is further drawn in the peripheral direction continuously from the second loop 21b while being folded alternately to the left side and to the right side in such a manner as to be woven together with the second loop 21b and the peripheral-direction unit 11 with a phase difference of 1/2 pitch from the second loop 21b, whereby a first loop 21a is formed. Thus, a peripheral-direction unit 21 in a first row (corresponding to the peripheral-direction unit 11) in which a plurality of mesh spaces are provided next to one another in the peripheral direction is formed (see Fig. 3M). The peripheral-direction unit 21 is shifted from the peripheral-direction unit 11 of the first woven structure 10 by 1/4 pitch in the peripheral direction and is woven together with the peripheral-direction unit 11.

Consequently, a second woven structure 20 is formed in which the peripheral-direction unit 21 in the first row, the peripheral-direction unit 22 in the second row, and the peripheral-direction unit 23 in the third row are arranged next to one another in the axial direction. Finally, the stent 100 according to the first embodiment illustrated in Fig. 2 is obtained.

In the stent 100 according to the first embodiment, the peripheral-direction units 11 to 13 of the first woven structure 10 have the same pitch length as one another and a phase difference of 1/4 pitch from one another, and so do the peripheral-direction units 21 to 23 of the second woven structure 20. Hence, the mesh spaces in the peripheral-direction units 11 to 13 of the first woven structure 10 can each be divided into four sections by the lines of the wire W2 forming the respective peripheral-direction units 21 to 23 of the second woven structure 20. Therefore, the mesh can be made finer than that of a stent including only the first woven structure 10.

In addition, the peripheral-direction units 21 to 23 of the second woven structure 20 are each shifted from a corresponding one of the peripheral-direction units 11 to 13 of the first woven structure 10 by 1/4 pitch in the peripheral direction. Furthermore, the bent parts of the peripheral-direction units 11 to 13 are not at the same peripheral-direction positions as the bent parts of the peripheral-direction units 21 to 23. Furthermore, the connections at the bent parts of the peripheral-direction units 11 to 13 of the first woven structure 10 (the connections each formed between each of the bent parts of one of the peripheral-direction units 11 to 13 and a corresponding one of the bent parts or the wire intersections of another peripheral-direction unit) are not provided at the same peripheral-direction positions as the connections at the bent parts of the peripheral-direction units 21 to 23 of the second woven structure 20 (the connections each formed between each of the bent parts of one of the peripheral-direction units 21 to 23 and a corresponding one of the bent parts or the wire intersections of another peripheral-direction unit). Hence, the deterioration in the followability along curved parts of the tubular organ can be avoided.

In addition, the peripheral-direction units 21 to 23 of the second woven structure 20 each have a smaller amplitude than a corresponding one of the peripheral-direction units 11 to 13 of the first woven structure 10. Therefore, the bent parts of the peripheral-direction units 11 to 13 are not at the same axial-direction positions as the bent parts of the peripheral-direction units 21 to 23. Furthermore, the connections at the bent parts of the peripheral-direction units 11 to 13 of the first woven structure 10 (the connections each formed between each of the bent parts of one of the peripheral-direction units 11 to 13 and a corresponding one of the bent parts or the wire intersections of another peripheral-direction unit) are not provided at the same axial-direction positions as the connections at the bent parts of the peripheral-direction units 21 to 23 of the second woven structure 20 (the connections each formed between each of the bent parts of one of the peripheral-direction units 21 to 23 and a corresponding one of the bent parts or the wire intersections of another peripheral-direction unit). Hence, the deterioration in the diameter reducibility of the stent can be avoided.

### <Second Embodiment>

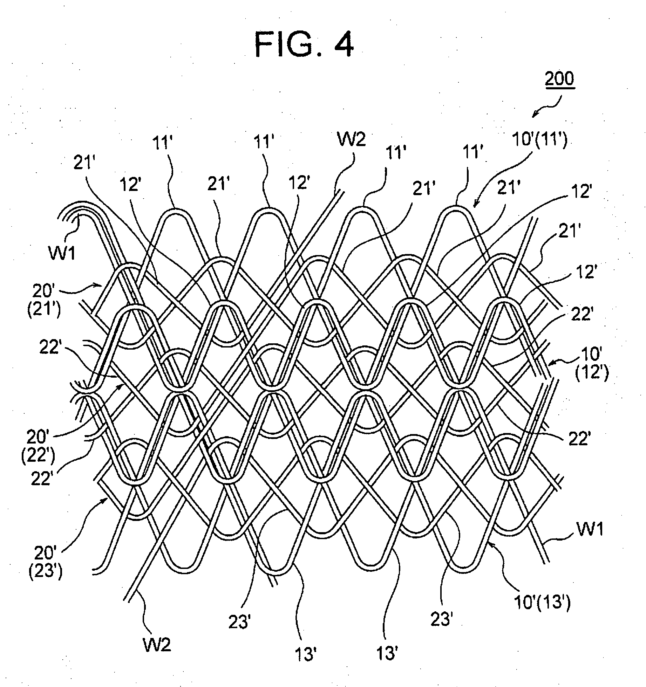

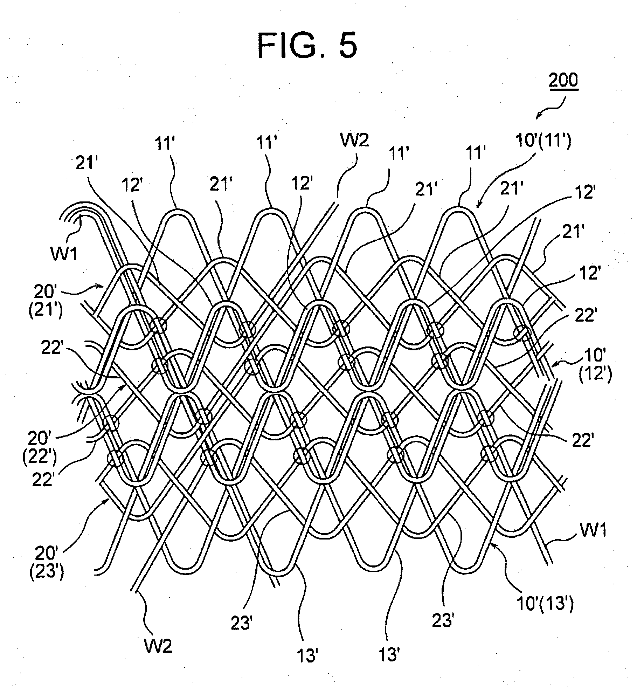

A stent 200 according to a second embodiment illustrated in Figs. 4 and 5 includes, as with the stent 100 according to the first embodiment, a first woven structure 10' including a peripheral-direction unit 11' in a first row, a peripheral-direction unit 12' in a second row, and a peripheral-direction unit 13' in a third row that are arranged next to one another in the axial direction; and a second woven structure 20' including a peripheral-direction unit 21' in a first row, a peripheral-direction unit 22' in a second row, and a peripheral-direction unit 23' in a third row that are arranged next to one another in the axial direction.

Fig. 5 illustrates the configuration illustrated in Fig. 4 with marks "o" given to differences from the configuration illustrated in Fig. 2 (among the intersections between the wire W1 forming the first woven structure and the wire W2 forming the second woven structure, intersections where the positions of the wire W1 and the wire W2 that are each either on the front side or on the back side are inverted from those illustrated in Fig. 2).

The first woven structure 10' included in the stent 200 according to the second embodiment has the same configuration as the first woven structure 10 included in the stent 100 according to the first embodiment.

The second woven structure 20' included in the stent 200 has the same configuration as the second woven structure 20 included in the stent 100 according to the first embodiment. However, the way the second woven structure 20' is woven together with the first woven structure 10' is different from the way the second woven structure 20 of the stent 100 is woven together with the first woven structure 10.

As illustrated in Fig. 4 (Fig. 5), in the stent 200 according to the second embodiment, the bent parts of the peripheral-direction unit 21' of the second woven structure 20' overlap but are not interlaced with the bent parts of the peripheral-direction unit 12' of the first woven structure 10'.

Furthermore, the bent parts of the peripheral-direction unit 22' of the second woven structure 20' overlap but are not interlaced with the bent parts of the peripheral-direction unit 11' of the first woven structure 10'.

Furthermore, the bent parts of the peripheral-direction unit 22' of the second woven structure 20' overlap but are not interlaced with the bent parts of the peripheral-direction unit 13' of the first woven structure 10'.

Furthermore, the bent parts of the peripheral-direction unit 23' of the second woven structure 20' overlap but are not interlaced with the bent parts of the peripheral-direction unit 12' of the first woven structure 10'.

The stent 200 according to the second embodiment produces the same advantageous effects as the stent 100 according to the first embodiment.

Moreover, as described above, the bent parts of each of the peripheral-direction units (21', 22', and 23') of the second woven structure 20' are not interlaced with the bent parts of any of the peripheral-direction units (12', 11' and 13', and 12') that is adjacent to one of the peripheral-direction units (11', 12', and 13') of the first woven structure 10' that corresponds to the peripheral-direction unit (21', 22', or 23'). Therefore, the first woven structure 10' and ultimately the stent 200 can be fully extended (the diameter thereof can be fully reduced). Accordingly, the diameter reducibility can be improved much more than in the case of the stent 100 according to the first embodiment including the interlaced parts.

### <Third Embodiment>

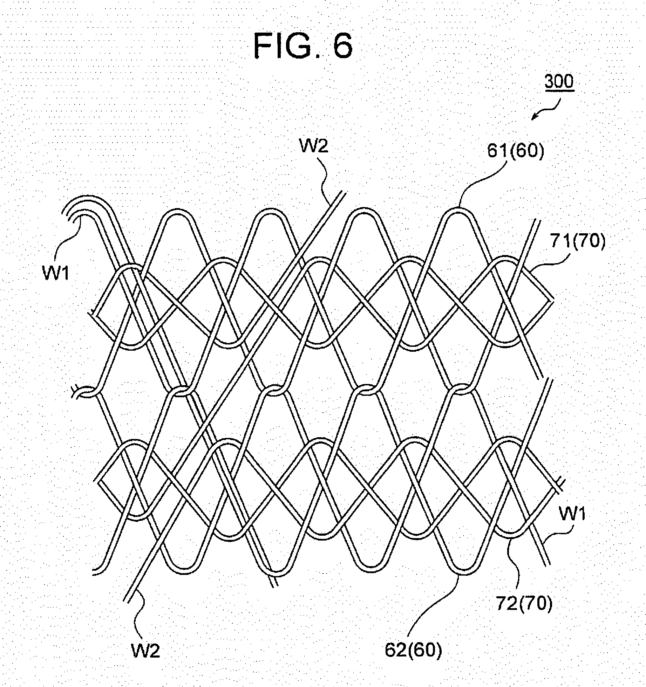

A stent 300 according to a third embodiment illustrated in Fig. 6 includes a first woven structure 60 in which a peripheral-direction unit 61 in a first row and a peripheral-direction unit 62 in a second row are arranged next to each other in the axial direction, and a second woven structure 70 in which a peripheral-direction unit 71 in a first row and a peripheral-direction unit 72 in a second row are arranged next to each other in the axial direction.

The peripheral-direction units (61, 62, 71, and 72) each include two loops. The two loops intersect each other at the linear parts thereof. One of the two loops runs alternately over and under the other loop.

As illustrated in Fig. 6, the peripheral-direction unit 61 in the first row and the peripheral-direction unit 62 in the second row included in the first woven structure 60 are shifted from each other by one pitch (amplitude) in the axial direction, and the bent parts of the peripheral-direction unit 61 are connected to (interlaced with) the bent parts of the peripheral-direction unit 62.

The peripheral-direction unit 71 in the first row of the second woven structure 70 has the same pitch length as the peripheral-direction unit 61 of the first woven structure 60 that corresponds thereto but a smaller amplitude than the peripheral-direction unit 61 and is woven together with the peripheral-direction unit 61 while being shifted from the peripheral-direction unit 61 by 1/4 pitch in the peripheral direction.

The peripheral-direction unit 71 has no parts overlapping the peripheral-direction unit 62 of the first woven structure 60. The bent parts of the peripheral-direction unit 71 are connected to neither the bent parts nor the wire intersections of the peripheral-direction unit 62.

The peripheral-direction unit 72 in the second row of the second woven structure 70 has the same pitch length as the peripheral-direction unit 62 of the first woven structure 60 that corresponds thereto but a smaller amplitude than the peripheral-direction unit 62. The peripheral-direction unit 72 is formed continuously from the end of the peripheral-direction unit 71 and is woven together with the peripheral-direction unit 62 while being shifted from the peripheral-direction unit 62 by 1/4 pitch in the peripheral direction.

The peripheral-direction unit 72 has no parts overlapping the peripheral-direction unit 61 of the first woven structure 60. The bent parts of the peripheral-direction unit 72 are connected to neither the bent parts nor the wire intersections of the peripheral-direction unit 61.

Furthermore, the peripheral-direction unit 72 has no parts overlapping the peripheral-direction unit 71 of the second woven structure 70. The bent parts of the peripheral-direction unit 72 are connected to neither the bent parts nor the wire intersections of the peripheral-direction unit 71.

In the stent 300 according to the third embodiment, the peripheral-direction units 61 and 62 of the first woven structure 60 have the same pitch length as each other and have a phase difference of 1/4 pitch from each other, and so do the peripheral-direction units 71 and 72 of the second woven structure 70. Hence, the mesh spaces in the peripheral-direction units 61 and 62 of the first woven structure 60 can each be divided into four sections by the lines of the wire W2 forming the respective peripheral-direction units 71 and 72 of the second woven structure 70. Therefore, the mesh can be made finer than that of a stent including only the first woven structure 60.

In addition, the peripheral-direction units 71 and 72 are each shifted from a corresponding one of the peripheral-direction units 61 and 62 of the first woven structure 60 by 1/4 pitch in the peripheral direction. Furthermore, the bent parts of the peripheral-direction units 61 and 62 are not at the same peripheral-direction positions as the bent parts of the peripheral-direction units 71 and 72. Furthermore, the connections at the bent parts of the peripheral-direction units 61 and 62 are not provided at the same peripheral-direction positions as the connections at the bent parts of the peripheral-direction units 71 and 72. Hence, the deterioration in the followability along curved parts of the tubular organ can be avoided.

In addition, the peripheral-direction units 71 and 72 each have a smaller amplitude than a corresponding one of the peripheral-direction units 61 and 62 of the first woven structure 60. Therefore, the bent parts of the peripheral-direction units 61 and 62 are not at the same axial-direction positions as the bent parts of the peripheral-direction units 71 and 72. Furthermore, the connections at the bent parts of the peripheral-direction units 61 and 62 are not provided at the same axial-direction positions as the connections at the bent parts of the peripheral-direction units 71 and 72. Hence, the deterioration in the diameter reducibility of the stent can be avoided.

### <Fourth Embodiment>

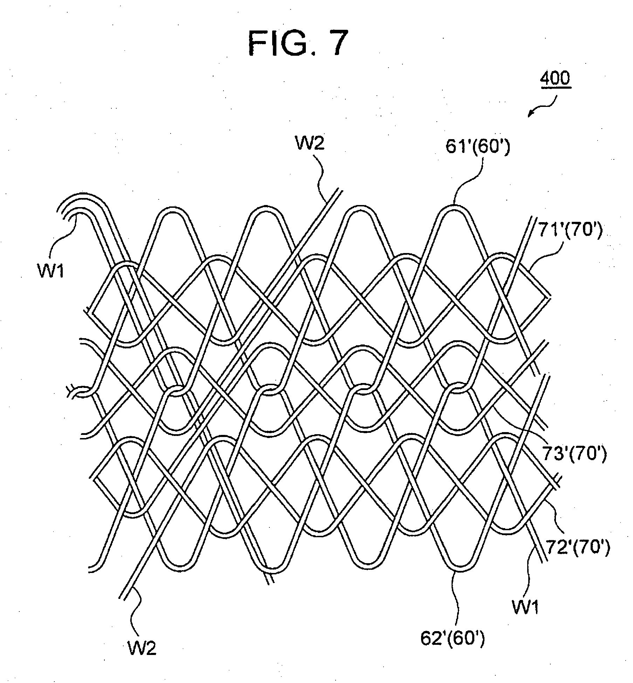

A stent 400 according to a fourth embodiment illustrated in Fig. 7 includes a first woven structure 60' in which a peripheral-direction unit 61' in a first row and a peripheral-direction unit 62' in a second row are arranged next to each other in the axial direction; and a second woven structure 70' in which a peripheral-direction unit 71', a peripheral-direction unit 73', and a peripheral-direction unit 72' are arranged next to one another in the axial direction.

The stent 400 is characterized in that the second woven structure 70' includes a peripheral-direction unit (the peripheral-direction unit 73') that corresponds to neither the peripheral-direction unit 61' nor the peripheral-direction unit 62' of the first woven structure 60'.

The first woven structure 60' included in the stent 400 according to the fourth embodiment has the same configuration as the first woven structure 60 included in the stent 300 according to the third embodiment.

The peripheral-direction unit 71' included in the second woven structure 70' has the same pitch length as the peripheral-direction unit 61' of the first woven structure 60' that corresponds thereto but a smaller amplitude than the peripheral-direction unit 61' and is woven together with the peripheral-direction unit 61' while being shifted from the peripheral-direction unit 61' by 1/4 pitch in the peripheral direction.

The peripheral-direction unit 71' has no parts overlapping the peripheral-direction unit 62' of the first woven structure 60'. The bent parts of the peripheral-direction unit 71' are connected to neither the bent parts nor the wire intersections of the peripheral-direction unit 62'.

The peripheral-direction unit 72' included in the second woven structure 70' has the same pitch length as the peripheral-direction unit 62' of the first woven structure 60' that corresponds thereto but a smaller amplitude than the peripheral-direction unit 62' and is woven together with the peripheral-direction unit 62' while being shifted from the peripheral-direction unit 62' by 1/4 pitch in the peripheral direction.

The peripheral-direction unit 72' has no parts overlapping the peripheral-direction unit 61' of the first woven structure 60'. The bent parts of the peripheral-direction unit 72' are connected to neither the bent parts nor the wire intersections of the peripheral-direction unit 61'.

The peripheral-direction unit 73' included in the second woven structure 70' has the same pitch length and the same amplitude (a smaller amplitude than the peripheral-direction units 61' and 62') as the peripheral-direction units 71' and 72'. The peripheral-direction unit 73' is provided between the peripheral-direction unit 71' and the peripheral-direction unit 72' and is woven together with the peripheral-direction unit 61' and the peripheral-direction unit 62'.

The bent parts of the peripheral-direction unit 73' are connected to (interlaced with) the bent parts of the peripheral-direction unit 61' of the first woven structure 60' and are also connected to (interlaced with) the bent parts of the peripheral-direction unit 62' of the first woven structure 60'.

The peripheral-direction unit 73' has no parts overlapping the peripheral-direction unit 71'. The bent parts of the peripheral-direction unit 73' are connected to neither the bent parts nor the wire intersections of the peripheral-direction unit 71'. Furthermore, the peripheral-direction unit 73' has no parts overlapping the peripheral-direction unit 72'. The bent parts of the peripheral-direction unit 73' are connected to neither the bent parts nor the wire intersections of the peripheral-direction unit 72'.

The stent 400 according to the fourth embodiment produces the same advantageous effects as the stent 300 according to the third embodiment.

Moreover, the mesh spaces defined by the peripheral-direction unit 61' and the peripheral-direction unit 62' of the first woven structure 60' can each be divided by the lines of the wire W2 forming the peripheral-direction unit 73' of the second woven structure 70'. Therefore, the mesh can be made much finer than that of the stent 300 according to the third embodiment that does not include the peripheral-direction unit 73'.

While some embodiments of the present invention have been described above, the present invention is not limited thereto and various changes can be made thereto. For example, the number of peripheral-direction units that are arranged next to one another in the axial direction in the first woven structure is not limited to three (Figs. 2 and 3) or two (Figs. 6 and 7) and may be 2 to about 20.

The stent according to the present invention can be used as a stent graft if at least a part of the inner periphery and/or the outer periphery is covered with a graft.

Such a graft may be any of grafts included in publicly known stent grafts. For example, the graft may be a cylindrical member made of thermoplastic resin shaped by a processing method such as extrusion molding or blow molding, a cylindrical textile fabric woven with thermoplastic resin fibers, a nonwoven cylindrical member made of thermoplastic resin, a cylindrical member made of a flexible resin sheet or porous sheet, or the like. The textile fabric may be any of known textile fabric or woven fabric such as plain-weave fabric and twill fabric. Moreover, the graft may have pleats formed by crimping or the like.

### Reference Signs List

- 100, 200, 300, 400: stent
- 10, 10': first woven structure
- 11, 11': peripheral-direction unit (first row)
- 11a: first loop
- 11b: second loop
- 12, 12': peripheral-direction unit (second row)
- 12a: first loop
- 12b: second loop
- 13, 13': peripheral-direction unit (third row)
- 13a: first loop
- 13b: second loop
- 20, 20': second woven structure
- 21, 21': peripheral-direction unit (first row)
- 21a: first loop
- 21b: second loop
- 22, 22': peripheral-direction unit (second row)
- 22a: first loop
- 22b: second loop
- 23, 23': peripheral-direction unit (third row)
- 23a: first loop
- 23b: second loop
- 60, 60': first woven structure
- 61, 61': peripheral-direction unit
- 62, 62': peripheral-direction unit
- 70, 70': second woven structure
- 71, 71': peripheral-direction unit
- 72, 72': peripheral-direction unit
- 73': peripheral-direction unit
- W1, W2: wire
- P1, P2: pitch length
- A1, A2: amplitude

## Claims

1. A stent formed with at least one wire woven into a cylindrical shape, the stent comprising:
a first woven structure including a plurality of peripheral-direction units that are arranged next to one another in an axial direction, the peripheral-direction units each having a plurality of mesh spaces provided next to one another in a peripheral direction, the peripheral-direction units each including a first loop and a second loop, the first loop having bent parts and linear parts formed by drawing the wire in the peripheral direction while folding the wire alternately to a left side and to a right side, the second loop having bent parts and linear parts formed by drawing the wire in the peripheral direction continuously from the first loop while folding the wire alternately to the left side and to the right side such that the second loop becomes out of phase with the first loop by 1/2 pitch; and
a second woven structure including a plurality of peripheral-direction units that are arranged next to one another in the axial direction in correspondence with the peripheral-direction units of the first woven structure, the peripheral-direction units each having a plurality of mesh spaces provided next to one another in the peripheral direction, the peripheral-direction units each including a first loop and a second loop, the first loop having bent parts and linear parts formed by drawing the wire in the peripheral direction while folding the wire alternately to the left side and to the right side at the same pitch length as the peripheral-direction units of the first woven structure and at a smaller amplitude than the peripheral-direction units of the first woven structure, the second loop having bent parts and linear parts formed by drawing the wire in the peripheral direction continuously from the first loop while folding the wire alternately to the left side and to the right side such that the second loop becomes out of phase with the first loop by 1/2 pitch,
wherein, in a region where each two of the peripheral-direction units of the first woven structure overlap each other, the bent parts of one of the two peripheral-direction units are connected to the bent parts or wire intersections of the other peripheral-direction unit,
wherein the peripheral-direction units of the second woven structure are each shifted from a corresponding one of the peripheral-direction units of the first woven structure by 1/4 pitch in the peripheral direction and are each woven together with the corresponding one of the peripheral-direction units of the first woven structure, and
wherein, regarding adjacent ones of the peripheral-direction units of the second woven structure, the bent parts of one of the adjacent peripheral-direction units are connected to neither the bent parts nor wire intersections of the other peripheral-direction unit.

2. The stent according to Claim 1,
wherein the plurality of peripheral-direction units of the first woven structure are arranged next to one another in the axial direction by straightly drawing, without bending, an end of the loop forming one of the peripheral-direction units and by forming a subsequent one of the peripheral-direction units at a position shifted from the preceding peripheral-direction unit in the axial direction, and
wherein the plurality of peripheral-direction units of the second woven structure are arranged next to one another in the axial direction by straightly drawing, without bending, an end of the loop forming one of the peripheral-direction units and by forming a subsequent one of the peripheral-direction units at a position shifted from the preceding peripheral-direction unit in the axial direction by an amount equal to an amount of shift between adjacent ones of the peripheral-direction units of the first woven structure.

3. The stent according to Claim 1 or 2, wherein the peripheral-direction units of the second woven structure have an amplitude of 25 to 95% of an amplitude of the peripheral-direction units of the first woven structure.

4. The stent according to any of Claims 1 to 3, wherein the peripheral-direction units of the first woven structure are each shifted from a preceding one of the peripheral-direction units by approximately 1/2 pitch in the axial direction and by approximately 1/4 pitch in the peripheral direction, and, in parts where adjacent ones of the peripheral-direction units of the first woven structure overlap each other, the bent parts of one of the adjacent peripheral-direction units are connected to the wire intersections of the other peripheral-direction unit.

5. The stent according to Claim 4, wherein the bent parts of each of the peripheral-direction units of the second woven structure are not connected to the bent parts of any peripheral-direction unit of the first woven structure that is adjacent to a corresponding one of the peripheral-direction units of the first woven structure.

6. A stent graft comprising: the stent according to any of Claims 1 to 5, and a graft that covers an inner periphery and/or an outer periphery of the stent.

## Patentansprüche

1. Stent, der aus wenigstens einem Draht besteht, der in eine zylindrische Form gewebt ist, wobei der Stent umfasst:
eine erste gewebte Struktur, die eine Vielzahl von Umfangsrichtungs-Einheiten enthält, die in einer axialen Richtung nebeneinander angeordnet sind, wobei die Umfangsrichtungs-Einheiten jeweils eine Vielzahl von Maschen-Räumen aufweisen, die sich in einer Umfangsrichtung nebeneinander befinden, die Umfangsrichtungs-Einheiten jeweils eine erste Schleife und eine zweite Schleife enthalten, die erste Schleife gebogene Teile und gerade Teile aufweist, die ausgebildet werden, indem der Draht in der Umfangsrichtung gezogen wird und dabei der Draht abwechselnd zu einer linken Seite und einer rechten Seite umgebogen wird, die zweite Schleife gebogene Teile und gerade Teile aufweist, die ausgebildet werden, indem der Draht in der Umfangsrichtung im Anschluss an die erste Schleife gezogen wird und dabei der Draht abwechselnd zu der linken Seite und der rechten Seite umgebogen wird, so dass die zweite Schleife zu der ersten Schleife um einen halben Schritt phasenversetzt wird; sowie
eine zweite gewebte Struktur, die eine Vielzahl von Umfangsrichtungs-Einheiten enthält, die in Entsprechung zu den Umfangsrichtungs-Einheiten der ersten gewebten Struktur in einer axialen Richtung nebeneinander angeordnet sind, wobei die Umfangsrichtungs-Einheiten jeweils eine Vielzahl von Maschen-Räumen aufweisen, die sich in der Umfangsrichtung nebeneinander befinden, die Umfangsrichtungs-Einheiten jeweils eine erste Schleife und eine zweite Schleife enthalten, die erste Schleife gebogene Teile und gerade Teile aufweist, die ausgebildet werden, indem der Draht in der Umfangsrichtung gezogen wird und dabei der Draht mit der gleichen Schrittlänge wie die Umfangsrichtungs-Einheiten der ersten gewebten Struktur und mit einer kleineren Amplitude als die Umfangsrichtungs-Einheiten der ersten gewebten Struktur abwechselnd zu der linken Seite und der rechten Seite umgebogen wird, die zweite Schleife gebogene Teile und gerade Teile aufweist, die ausgebildet werden, indem der Draht in der Umfangsrichtung im Anschluss an die erste Schleife gezogen wird und dabei der Draht abwechselnd zu der linken Seite und der rechten Seite umgebogen wird, so dass die zweite Schleife zu der ersten Schleife um einen halben Schritt phasenversetzt wird;
wobei in einem Bereich, in dem jeweils zwei der Umfangsrichtungs-Einheiten der ersten gewebten Struktur einander überlappen, die gebogenen Teile einer der zwei UmfangsrichtungsEinheiten mit den gebogenen Teilen oder Draht-Überkreuzungen der anderen Umfangsrichtungs-Einheit verbunden sind,
die Umfangsrichtungs-Einheiten der zweiten gewebten Struktur jeweils gegenüber einer entsprechenden der Umfangsrichtungs-Einheiten der ersten gewebten Struktur um einen viertel Schritt in der Umfangsrichtung versetzt sind und jeweils mit der entsprechenden einen der Umfangsrichtungs-Einheiten der ersten gewebten Struktur verwebt sind, und
hinsichtlich benachbarter der Umfangsrichtungs-Einheiten der zweiten gewebten Struktur die gebogenen Teile einer der angrenzenden Umfangsrichtungs-Einheiten weder mit den gebogenen Teilen noch mit Draht-Überkreuzungen der anderen Umfangsrichtungs-Einheit verbunden sind.

2. Stent nach Anspruch 1,
wobei die Vielzahl von Umfangsrichtungs-Einheiten der ersten gewebten Struktur in der axialen Richtung nebeneinander angeordnet werden, indem ein Ende der Schleife, das ein Ende der Umfangsrichtungs-Einheiten bildet, ohne Biegen gerade gezogen wird und eine nachfolgende der Umfangsrichtungs-Einheiten an einer Position ausgebildet wird, die gegenüber der vorhergehenden Umfangsrichtungs-Einheit in der axialen Richtung versetzt ist, und
wobei die Vielzahl von Umfangsrichtungs-Einheiten der zweiten gewebten Struktur in der axialen Richtung nebeneinander angeordnet werden, indem ein Ende der Schleife, das ein Ende der Umfangsrichtungs-Einheiten bildet, ohne Biegen gerade gezogen wird und eine nachfolgende der Umfangsrichtungs-Einheiten an einer Position ausgebildet wird, die gegenüber der vorhergehenden Umfangsrichtungs-Einheit in der axialen Richtung um ein Maß verschoben ist, das einem Maß der Verschiebung benachbarter der Umfangsrichtungs-Einheiten der ersten gewebten Struktur zueinander gleich ist.

3. Stent nach Anspruch 1 oder 2, wobei die Umfangsrichtungs-Einheiten der zweiten gewebten Struktur eine Amplitude von 25 bis 95 % einer Amplitude der Umfangsrichtungs-Einheiten der ersten gewebten Struktur haben.

4. Stent nach einem der Ansprüche 1 bis 3, wobei die Umfangsrichtungs-Einheiten der ersten gewebten Struktur jeweils um ungefähr einen halben Schritt in der axialen Richtung und um ungefähr einen viertel Schritt in der Umfangsrichtung gegenüber einer vorhergehenden der Umfangsrichtungs-Einheiten versetzt sind und in Teilen, in denen benachbarte der Umfangsrichtungs-Einheiten der ersten gewebten Struktur einander überlappen, die gebogenen Teile einer der benachbarten Umfangsrichtungs-Einheiten mit den Draht-Überkreuzungen der anderen Umfangsrichtungs-Einheit verbunden sind.

5. Stent nach Anspruch 4, wobei die gebogenen Teile jeder der Umfangsrichtungs-Einheiten der zweiten gewebten Struktur nicht mit den gebogenen Teilen einer Umfangsrichtungs-Einheit der ersten gewebten Struktur verbunden sind, die an eine entsprechende der Umfangsrichtungs-Einheiten der ersten gewebten Struktur angrenzt.

6. Stentgraft, der den Stent nach einem der Ansprüche 1 bis 5 sowie einen Graft umfasst, der einen Innenumfang und/oder einen Außenumfang des Stents abdeckt.

## Revendications

1. Endoprothèse formée d'au moins un fil tissé en une forme cylindrique, l'endoprothèse comprenant :
une première structure tissée comprenant une pluralité de motifs dans le sens de la périphérie qui sont disposés l'un près de l'autre dans un sens axial, les motifs dans le sens de la périphérie ayant chacun une pluralité d'espaces maillés disposés l'un près de l'autre dans un sens périphérique, les motifs dans le sens de la périphérie comprenant chacun une première boucle et une seconde boucle, la première boucle ayant des parties fléchies et des parties linéaires formées par étirage du fil dans le sens périphérique tout en pliant le fil alternativement vers un côté gauche et vers un côté droit, la seconde boucle ayant des parties fléchies et des parties linéaires formées par étirage du fil dans le sens périphérique continuellement depuis la première boucle tout en pliant le fil alternativement vers le côté gauche et vers le côté droit de sorte que la seconde boucle est déphasée par rapport à la première boucle d'un demi pas ; et
une seconde structure tissée comprenant une pluralité de motifs dans le sens de la périphérie qui sont disposés l'un près de l'autre dans le sens axial en correspondance avec les motifs dans le sens de la périphérie de la première structure tissée, les motifs dans le sens de la périphérie ayant chacun une pluralité d'espaces maillés disposés l'un près de l'autre dans le sens périphérique, les motifs dans le sens de la périphérie comprenant chacun une première boucle et une seconde boucle, la première boucle ayant des parties fléchies et des parties linéaires formées par étirage du fil dans le sens périphérique tout en pliant le fil alternativement vers le côté gauche et vers le côté droit à la même longueur de pas que les motifs dans le sens de la périphérie de la première structure tissée et à une amplitude inférieure aux motifs du sens périphérique de la première structure tissée, la seconde boucle ayant des parties fléchies et des parties linéaires formées par étirage du fil dans le sens de la périphérie continuellement depuis la première boucle tout en pliant le fil alternativement vers le côté gauche et vers le côté droit de sorte que la seconde boucle est déphasée par rapport à la première boucle d'un demi pas,
où, dans une région où deux de chaque motif dans le sens de la périphérie de la première structure tissée se chevauchent l'un l'autre, les parties fléchies de l'un des deux motifs dans le sens de la périphérie sont reliées aux parties fléchies ou aux intersections de fil de l'autre motif du sens de la périphérie,
où les motifs dans le sens de la périphérie de la seconde structure tissée sont chacun décalés depuis l'un correspondant des motifs de sens périphérique de la première structure tissée d'un quart de pas dans le sens de la périphérie et sont chacun tissés ensemble avec celui correspondant des motifs de sens périphérique de la première structure tissée, et
où, concernant ceux adjacents des motifs du sens périphérique de la seconde structure tissée, les parties fléchies de l'un des motifs adjacents de sens périphérique ne sont reliées ni aux parties fléchies ni aux intersections de fil de l'autre motif de sens périphérique.

2. Endoprothèse selon la revendication 1,
dans laquelle la pluralité des motifs de sens périphérique de la première structure tissée sont disposés l'un près de l'autre dans le sens axial par étirage de manière droite, sans flexion, d'une extrémité de la boucle formant l'un des motifs du sens périphérique et en formant l'un ultérieur des motifs de sens périphérique à une position décalée depuis le motif précédent de sens périphérique dans le sens axial, et
où la pluralité des motifs de sens périphérique de la seconde structure tissée sont disposés l'un près de l'autre dans le sens axial par étirage de manière droite, sans flexion, une extrémité de la boucle formant l'un des motifs de sens périphérique et en formant l'un ultérieur des motifs de sens périphérique à une position décalée depuis le motif de sens périphérique précédent dans le sens axial d'une quantité égale à une quantité de décalage entre ceux adjacents des motifs de sens périphérique de la première structure tissée.

3. Endoprothèse selon la revendication 1 ou 2, dans laquelle les motifs de sens périphérique de la seconde structure tissée présentent une amplitude de 25 à 95 % d'une amplitude des motifs de sens périphérique de la première structure tissée.

4. Endoprothèse selon l'une quelconque des revendications 1 à 3, dans laquelle les motifs de sens périphérique de la première structure tissée sont chacun décalés de l'un précédent des motifs de sens périphérique d'approximativement 1/2 pas dans le sens axial et d'approximativement 1/4 de pas dans le sens périphérique, et, dans les parties où ceux adjacents des motifs de sens périphérique de la première structure tissée se chevauchent l'un à l'autre, les parties fléchies de l'un des motifs adjacents de sens périphérique sont reliées aux intersections du fil de l'autre motif de sens périphérique.

5. Endoprothèse selon la revendication 4, dans laquelle les parties fléchies de chacun des motifs de sens périphérique de la seconde structure tissée ne sont pas reliées aux parties fléchies de n'importe quel motif de sens périphérique de la première structure tissée qui est adjacente à l'un correspondant des motifs de sens périphérique de la première structure tissée.

6. Greffe d'endoprothèse comprenant : l'endoprothèse selon l'une quelconque des revendications 1 à 5, et une greffe qui recouvre une périphérie interne et/ou une périphérie externe de l'endoprothèse.
